# EUROPÄISCHE PATENTSCHRIFT

(11) **EP 1 511 720 B1**
(45) Veröffentlichungstag und Bekanntmachung des Hinweises auf die Patenterteilung: **09.08.2006**
(21) Anmeldenummer: 03755046.4
(22) Anmeldetag: 21.05.2003
(51) Int. Cl.: C07C 253/34, C07C 209/84

(54) **VERFAHREN ZUR REDUZIERUNG DES GEHALTS AN EINEM UNGESÄTTIGTEN AMIN IN EINER MISCHUNG ENTHALTEND EIN AMINONITRIL, EIN DIAMIN, EIN DINITRIL ODER DEREN GEMISCHE**
METHOD FOR REDUCING THE CONTENT OF AN UNSATURATED AMINE IN A MIXTURE CONTAINING AN AMINO NITRILE, A DIAMINE, A DINITRILE OR MIXTURES THEREOF
PROCEDE DE REDUCTION DE LA TENEUR EN AMINE INSATUREE D'UN MELANGE CONTENANT AMINONITRILE, DIAMINE, DINITRILE OU DES MELANGES DE CEUX-CI

(30) Priorität: 28.05.2002 DE 10223827
(43) Veröffentlichungstag der Anmeldung: 09.03.2005
(73) Patentinhaber: BASF Aktiengesellschaft, 67056 Ludwigshafen (DE)
(72) Erfinder: MAIXNER, Stefan, 68723 Schwetzingen (DE); BENISCH, Christoph, 68165 Mannheim (DE); LUYKEN, Hermann, 67069 Ludwigshafen (DE); BASSLER, Peter, 68519 Viernheim (DE); FISCHER, Rolf-Hartmuth, 69121 Heidelberg (DE); MELDER, Johann-Peter, 67459 Böhl-Iggelheim (DE); ANSMANN, Andreas, 69168 Wiesloch (DE)
(86) Internationale Anmeldenummer: PCT/EP2003/005313
(87) Internationale Veröffentlichungsnummer: WO 2003/099768

(56) Entgegenhaltungen:
- EP-A- 0 497 333
- EP-A- 0 502 439
- WO-A-02/44135
- WO-A-93/14064
- WO-A-93/16984
- WO-A-98/34900
- WO-A-98/34912
- KOSKIKALLIO J: "NUCLEOPHILIC REACTIVITY PART II. KINETICS OF REACTIONS OF METHYL PERCHLORATE WITH NUCLEOPHILES IN WATER" ACTA CHEMICA SCANDINAVICA, MUNKSGAARD, COPENHAGEN, DK, Bd. 23, Nr. 5, 1969, Seiten 1477-1489, XP001064740 ISSN: 0904-213X in der Anmeldung erwähnt

## Beschreibung

Die vorliegende Erfindung betrifft ein Verfahren zur Reduzierung des Gehalts an einem aliphatischen, einfach ungesättigten Amin (IV) in einer Mischung (V) enthaltend ein aliphatisches Aminonitril (I) mit 4 bis 12 C-Atomen oder ein aliphatisches Diamin (II) mit 4 bis 12 C-Atomen oder ein aliphatisches Dinitril (III) mit 4 bis 12 C-Atomen oder deren Gemische und Amin (IV), wobei man
a) Mischung (V) mit einem anionischen Nukleophil (VI),
   das ein nukleophiles Atom ausgewählt aus der Gruppe bestehend aus
   Sauerstoff, Stickstoff und Schwefel aufweist,
   das zur Aufnahme eines H⁺-Ions fähig ist unter Bildung einer Säure mit einem pKₐ-Wert im Bereich von 7 bis 11, gemessen in Wasser bei 25°C, und
   das eine relative Nukleophilie, gemessen in Methylperchlorat/Methanol bei 25°C, im Bereich von 3,4 bis 4,7 im Falle von Sauerstoff als nukleophilem Atom,
   im Bereich von 4,5 bis 5,8 im Falle von Stickstoff als nukleophilem Atom und
   im Bereich von 5,5 bis 6,8 im Falle von Schwefel als nukleophilem Atom
   aufweist,
   in einer Menge im Bereich von 0,01 bis 10 Mol pro Mol an Amin (IV) in Mischung (V) bei einer Temperatur im Bereich von 50 bis 200°C unter Erhalt einer Mischung (VII) umsetzt,
b) Aminonitril (I) oder Diamin (II) oder Dinitril (III) oder deren Gemische von Mischung (VII) bei einer Temperatur im Bereich von 50 bis 200°C und einem Druck im Bereich von 0,1 bis 100 kPa abdestilliert unter Erhalt eines Sumpfproduktes (VIII),
   dadurch gekennzeichnet, dass man
c) von Sumpfprodukt (VIII) ein Aminonitril (I) oder Diamin (II) oder Dinitril (III) oder deren Gemische bei einer Temperatur, die niedriger ist als die in Schritt b) gewählte abdestilliert.

Mischungen enthaltend ein Aminonitril, ein Diamin, ein Dinitril oder deren Gemische und ein ungesättigtes Amin, wobei im Sinne der vorliegenden Erfindung unter einem ungesättigten Amin eine zyklische oder lineare Verbindung enthaltend mindestens eine Kohlenstoff-Stickstoff-Doppelbindung oder eine Verbindung, die mindestens eine Kohlenstoff-Stickstoff-Doppelbindung, beispielsweise durch eine Eliminierungsreaktion, bilden kann, verstanden wird, fallen üblicherweise bei der partiellen Hydrierung von Dinitrilen zu Aminonitrilen oder einem Gemisch aus Aminonitrilen und Diaminen oder bei der vollständigen Hydrierung von Dinitrilen zu Diaminen an.

Die partielle Hydrierung von Adipodinitril (ADN) unter gleichzeitiger Herstellung von Hexamethylendiamin (HMD) und 6-Aminocapronitril (ACN), sowie die vollständige Hydrierung von ADN zu HMD, in Gegenwart eines Katalysators auf der Basis eines Metalls wie Nickel, Cobalt, Eisen, Rhodium oder Ruthenium ist allgemein bekannt, beispielsweiwe aus: K.Weissermel, H.-J.Arpe, Industrielle Organische Chemie, 3.Auflage, VCH Verlagsgesellschaft mbH, Weinheim, 1988, Seite 266, US-A 4 601 859, US-A 2 762 835, US-A 2 208 598, DE-A 848 654, DE-A 954 416, DE-A 42 35 466, US-A 3 696 153, DE-A 19500222, WO-A-92/21650 und DE-A-19548289.

Als Nebenprodukte entstehen unter anderem Azepin-Derivate wie N-(2-Azepano)-1,6-Diaminohexan und N-(2-Azepano)-6-Aminocapronitril, insbesondere 2-Aminoazepan und Tetrahydroazepin (THA).

Diese Azepin-Derivate, die wegen ihrer Farbgebung und Verschlechterung der Produkteigenschaften unerwünschte Verunreinigungen der üblicherweise für die Herstellung von Kunstfasern oder technischen Kunststoffen verwendeten Aminonitrile und Diamine darstellen, lassen sich nur mit erheblichem Aufwand von den Aminonitrilen, Diaminen oder deren Gemischen abtrennen.

Aus der WO-A-98/34900 und WO-A-98/34912 sind Verfahren zur Reduzierung der Gehalte an Tetrahydroazepin in einer Mischung enthaltend 6-Aminocapronitril bekannt.

EP-A-497333 beschreibt die Trennung von aliphatischen Aminonitrilen oder aliphatischen Diaminen aus Mischungen, die ein aliphatisches Aminonitril oder aliphatisches Diamin und ein zyklisches, aliphatisches, einfach ungesättigtes Amin enthalten, durch Zugabe von Basen, wobei die Base im stöchiometrischen Überschuß bezogen auf das zyklische, aliphatische, einfach ungesättigte Amin einzusetzen ist. Dabei werden als Basen Alkalihydroxide, Erdalkalihydroxide, Tetraalkylammoniumhydroxid, Alkalialkoxide und Erdalkalialkoxide empfohlen.

Nachteilig bei diesem Verfahren ist eine gleichzeitig stattfindende Polymerisation von Wertprodukt, die zu einem erheblichen Verlust an Wertprodukt und zu unerwünschten Ablagerungen in den zu der Ausübung des Verfahrens eingesetzten Apparaturen und Maschinen führt.

Die zum Zeitpunkt der vorliegenden Anmeldung noch nicht offengelegte Anmeldung PCT/EP01/13954 beschreibt die Reduzierung des Gehalts an einem aliphatischen einfach ungesättigten Amin in einer Mischung enthaltend ein Aminonitril, ein Diamin oder deren Gemische und dieses Amin entsprechend den Schritten a) und b) des vorliegenden Verfahrens. Das in Schritt b) erhaltene Sumpfprodukt wird in dem beschriebenen Verfahren verworfen.

Aufgabe der vorliegenden Erfindung war es daher, ein Verfahren bereitzustellen, das die Reduzierung des Gehalts an einem aliphatischen, einfach ungesättigten Amin in einer Mischung, enthaltend ein Aminonitril oder Diamin oder deren Gemische und ein aliphatisches, einfach ungesättigtes Amin, auf technisch einfache und wirtschaftliche Weise mit höherer Ausbeute an Wertprodukt unter Vermeidung der genannten Nachteile ermöglicht.

Überraschenderweise wurde gefunden, dass gemäß eingangs definiertem Verfahren aus dem bereits durch Destillation in Schritt b) erhaltenen Sumpfprodukt durch eine weitere Destillation gemäß Schritt c) weiteres Aminonitril (I), Diamin (II), Dinitril (III) oder deren Gemische erhalten werden kann.

Als Aminonitril (I) kommen Verbindungen mit 4 bis 12 C-Atomen und einer oder mehreren, wie zwei, drei oder vier, vorzugsweise einer Nitrilgruppe in Betracht, insbesondere solche, die mindestens eine Nitrilgruppe aufweisen, die in Nachbarschaft zu einem aliphatischen Kohlenstoffatom steht, welches einen oder zwei, vorzugsweise zwei Wasserstoffatome trägt, oder Gemische solcher Aminonitrile.

Als Aminonitril (I) kommen Verbindungen mit 4 bis 12 C-Atomen und einer oder mehreren, wie zwei, drei oder vier, vorzugsweise einer Aminogruppe in Betracht, insbesondere solche, die mindestens eine Aminogruppe aufweisen, die in Nachbarschaft zu einem aliphatischen Kohlenstoffatom steht, welches einen oder zwei, vorzugsweise zwei Wasserstoffatome trägt, oder Gemische solcher Aminonitrile. Besonders bevorzugt sind Aminonitrile mit endständiger, also am Ende einer Alkylkette befindlichen, Aminogruppe.

Als Aminonitril (I) kommen vorzugsweise Aminonitrile ausgewählt aus der Gruppe bestehend aus 4-Aminobutyronitril, 5-Aminovaleronitril, 2-Methyl-5-aminovaleronitril, 6-Aminocapronitril, 12-Aminododecanitril, insbesondere 6-Aminocapronitril, in Betracht.

Die Herstellung solcher Aminonitrile kann in an sich bekannter Weise erfolgen.

6-Aminocapronitril kann durch partielle katalytische Hydrierung mit einem molekularen Wasserstoff enthaltenden Gas ausgehend von ADN zu Mischungen, die HMD und ACN enthalten, erhalten werden. Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannte Umsetzung sind beispielsweise in WO-A-96/20166, DE-A-19636768 und DE-A-19646436 beschrieben.

Als Diamin (II) kommen Verbindungen mit 4 bis 12 KohlenstoffAtome und zwei oder mehr, wie zwei, drei oder vier, vorzugsweise zwei Aminogruppen in Betracht, insbesondere solche, die mindestens zwei Aminogruppen aufweisen, die in Nachbarschaft zu einem aliphatischen Kohlenstoffatom stehen, welches ein oder zwei, vorzugsweise zwei Wasserstoffatome trägt, besonders bevorzugt Diamine mit endständigen, also am Ende einer Alkylkette befindlichen Aminogruppen oder Gemische solcher Diamine.

Als Diamin (II) kommen vorzugsweise Diamine ausgewählt aus der Gruppe bestehend aus 1,4-Diaminobutan, 1,5-Diaminopentan, 2-Methyl-1,5-diaminopentan, 1,6-Diaminohexan (HMD), 1,12-Diaminododecan in Betracht.

Die Herstellung solcher Diamine kann in an sich bekannter Weise erfolgen.

HMD kann man durch partielle katalytische Hydrierung mit einem molekularen Wasserstoff enthaltenden Gas ausgehend von ADN zu Mischungen, die HMD und ACN enthalten, oder durch vollständige Hydrierung mit einem molekularen Wasserstoff enthaltenden Gas von ADN erhalten werden.

Als Katalysatoren können bei dieser Hydrierung vorteilhaft solche auf der Basis eines Metalles ausgewählt aus der Gruppe bestehend aus Ruthenium, Rhodium, Nickel, Cobalt, vorzugsweise Eisen eingesetzt werden, wobei die Katalysatoren weitere Elemente als Promotoren enthalten können. Im Falle von Katalysatoren auf der Basis von Eisen kommen als Promotoren insbesondere ein oder mehrere, wie zwei, drei, vier oder fünf Elemente ausgewählt aus der Gruppe bestehend aus Aluminium, Silizium, Zirkonium, Titan und Vanadium in Betracht.

Solche Katalysatoren sowie die Verfahrensbedingungen für die genannten Umsetzungen können beispielsweise den bereits oben genannten Veröffentlichungen entnommen werden.

Als Dinitril (III) kommen Verbindungen mit 4 bis 12 Kohlenstoff-Atomen und zwei oder mehr, wie zwei, drei oder vier, vorzugsweise zwei Nitrilgruppen in Betracht, insbesondere solche, die mindestens zwei Nitrilgruppen aufweisen, die in Nachbarschaft zu einem aliphatischen Kohlenstoffatom stehen, welches ein oder zwei, vorzugsweise zwei Wasserstoffatome trägt, besonders bevorzugt Dinitrile mit endständigen, also am Ende einer Alkylkette befindlichen Nitrilgruppen oder Gemische solcher Dinitrile.

Als Dinitril (III) kommen solche mit 4 bis 12 Kohlenstoff-Atomen vorzugsweise Dinitrile ausgewählt aus der Gruppe bestehend aus 1,2-Dinitriloethan, 1,3-Dinitrilopropan, 1-Methyl-1,3-dinitrilopropan, 1,4-Dinitrilobutan, 1,10-Dinitrilodecan in Betracht.

Die Herstellung solcher Dinitrile kann in an sich bekannter Weise erfolgen.

So kann beispielsweise die Herstellung von ADN durch doppelte Hydrocyanierung von Butadien in Gegenwart von Nickel und Phosphorhaltigen Liganden erfolgen. Beispiele für geeignete Katalysatoren sowie Verfahrensbedingungen für die genannten Umsetzungen können beispielsweise den bereits oben genannten Veröffentlichungen entnommen werden.

Als Amine (IV) kommen zyklische oder lineare Verbindungen enthaltend mindestens eine Kohlenstoff-Stickstoff-Doppelbindung oder eine Verbindung, die mindestens eine Kohlenstoff-Stickstoff-Doppelbindung, beispielsweise durch eine Eliminierungsreaktion, bilden kann, oder Gemische solcher Verbindungen in Betracht.

Insbesondere ist Amin (IV) eine Verbindung der Formel

R¹-(CH₂)ₙ-CH=N-(CH₂)ₘ-R²

wobei
- n, m: unabhängig voneinander 1, 2, 3, 4,5, 6, 7, 8, 9, 10, 11. oder 12
vorzugsweise 4, 5 oder 6 und
- R¹, R²: unabhängig voneinander -CN oder -NH₂ darstellen
oder der Formel 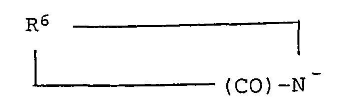 wobei
- R³: einen Alkenylrest mit 3, 4, 5, 6, 7, 8, 9, 10, 11 zu dem Ringsystem gehörigen Kohlenstoffatomen darstellt

Ganz insbesondere kommt als Amin (IV) eine Verbindung ausgewählt aus der Gruppe bestehend aus, Dihydropyrrol, Tetrahydropyridin, 3-Methyl-Tetrahydropyridin, Tetrahydroazepin, Mono-ungesättigte Cyclododecylamine oder deren Gemische in Betracht.

Diese Amine (IV) können in der Mischung (V) als einzelne Verbindungen oder als Addukte, beispielsweise an ein Nitril (I), insbesondere Aminonitril, vorliegen, wobei diese Addukte im Sinne der vorliegenden Erfindung ebenfalls als Amin (IV) bezeichnet werden.

Solche Amine (IV) sowie Verfahren zu deren Herstellung sind allgemein bekannt.

So kann Tetrahydroazepin bei der partiellen katalytischen Hydrierung mit einem molekularen Wasserstoff enthaltenden Gas ausgehend von ADN zu Mischungen, die HMD und ACN enthalten, in der Regel in Mengen von 1 bis 10000 ppm bezogen auf das Gemisch nach den für die Herstellung der ACN beschriebenen Verfahren in Mischungen (V) erhalten werden.

Ebenso können die genannten Amine (IV) durch Oxidation von Aminen, wie HMD, beispielsweise mit molekularen Sauerstoff enthaltenden Gasen, gebildet werden.

In einer bevorzugten Ausführungsform kann man als Mischung (V) den aus der partiellen katalytischen Hydrierung, wie Gasphasenhydrierung oder Flüssigphasenhydrierung, mit einem molekularen Wasserstoff enthaltenden Gas ausgehend von Dinitrilen, insbesondere ADN, in Gegenwart eines Katalysators, wie eines Suspensionskatalysators oder Festbettkatalysators, erhaltenen Reaktionsaustrag, der im Falle von ADN als Ausgangsverbindung ACN als Aminonitril (I), HMD als Diamin (II), Rest-ADN als Dintril (III) und Tetrahydroazepin als Amin (IV) enthält, einsetzen, wobei gewünschtenfalls zuvor bei der Hydrierung gegebenenfalls eingesetztes Lösungsmittel teilweise oder vollständig abgetrennt werden kann. Nach den bisherigen Beobachtungen kann es vorteilhaft sein, einen bei der Hydrierung eingesetzten Katalysator vor dem Einsatz von Mischung (V) in dem erfindungsgemäßen Verfahren abzutrennen.

Vorteilhaft kann man von dem Gemisch, enthaltend Aminonitril (I), Diamin (II), Dinitril (III) und Amin (IV), wobei insbesondere im Falle von ADN als Ausgangsverbindung ACN als Aminonitril (I), HMD als Diamin (II), Rest-ADN als Dinitril (III) und Tetrahydroazepin als Amin (IV) in Betracht kommen, vor Schritt a) das Diamin (I) nach an sich bekannten Verfahren, wie durch Destillation abtrennen. In diesem Fall kommt als in Schritt a) eingesetzte Mischung (V) ein Gemisch enthaltend Aminonitril (I), Dinitril (III) und Amin (IV), insbesondere im Falle von ADN als Ausgangsverbindung ACN als Aminonitril (I), Rest-ADN als Dintril (III) und Tetrahydroazepin als Amin (IV), in Betracht.

Vorteilhaft kann man von dem Gemisch, enthaltend Aminonitril (I), Diamin (II), Dinitril (III) und Amin (IV), wobei insbesondere im Falle von ADN als Ausgangsverbindung ACN als Aminonitril (I), HMD als Diamin (II), Rest-ADN als Dintril (III) und Tetrahydroazepin als Amin (IV) in Betracht kommen, vor Schritt a) das Dinitril (III) nach an sich bekannten Verfahren, wie durch Destillation abtrennen. In diesem Fall kommt als in Schritt a) eingesetzte Mischung (V) ein Gemisch enthaltend Aminonitril (I), Diamin(II) und Amin (IV), insbesondere im Falle von ADN als Ausgangsverbindung ACN als Aminonitril (I), / HMD als Diamin (II) und Tetrahydroazepin als Amin (IV), in Betracht.

Vorteilhaft kann man von dem Gemisch, enthaltend Aminonitril (I), Diamin (II), Dinitril (III) und Amin (IV), wobei insbesondere im Falle von ADN als Ausgangsverbindung ACN als Aminonitril (I), HMD als Diamin (II), Rest-ADN als Dintril (III) und Tetrahydroazepin als Amin (IV) in Betracht kommen, vor Schritt a) das Dinitril (III) und das Diamin (II) nach an sich bekannten Verfahren, wie durch Destillation abtrennen. In diesem Fall kommt als in Schritt a) eingesetzte Mischung (V) ein Gemisch enthaltend Aminonitril (I) und Amin (IV), insbesondere im Falle von ADN als Ausgangsverbindung ACN als Aminonitril (I) und Tetrahydroazepin als Amin (IV), in Betracht.

In einer bevorzugten Ausführungsform kann man als Mischung (V) den aus der vollständigen katalytischen Hydrierung, wie Gasphasenhydrierung oder Flüssigphasenhydrierung, mit einem molekularen Wasserstoff enthaltenden Gas ausgehend von Dinitrilen, insbesondere ADN, in Gegenwart eines Katalysators, wie eines Suspensionskatalysators oder. Festbettkatalysators, erhaltenen Reaktionsaustrag, der im Falle von ADN als Ausgangsverbindung HMD als Diamin (II) und Tetrahydroazepin als Amin (IV) enthält, einsetzen, wobei gewünschtenfalls zuvor bei der Hydrierung gegebenenfalls eingesetztes Lösungsmittel teilweise oder vollständig abgetrennt werden kann. Nach den bisherigen Beobachtungen kann es vorteilhaft sein, einen bei der Hydrierung eingesetzten Katalysator vor dem Einsatz von Mischung (V) in dem erfindungsgemäßen Verfahren abzutrennen.

Erfindungsgemäß versetzt man Mischung (V) mit einem anionischen Nukleophil (VI) .

Unter dem Begriff anionisch wird im Sinne der vorliegenden Erfindung verstanden, daß Nukleophil (VI) in Summe einfach oder mehrfach, wie zwei oder dreifach, vorzugsweise einfach negativ geladen ist.

Unter dem Begriff nukleophil wird im Sinne der vorliegenden Erfindung die in Koskikallo, Acta Chem. Scand. 23 (1969) Seiten 1477-1489 beschriebene Fähigkeit einer Verbindung verstanden, in methanolischer Lösung aus Methylperchlorat bei 25°C die Perchlorat-Gruppe zu verdrängen, wobei die verbleibende Methylgruppe über ein nukleophiles Atom von Verbindung (VI) an Verbindung (VI) gebunden wird.

Als nukleophiles Atom von Verbindung (VI) kommt ein Atom ausgewählt aus der Gruppe bestehend aus Stickstoff, Sauerstoff und Schwefel, vorzugsweise Stickstoff oder Sauerstoff in Betracht.

Erfindungsgemäß ist Verbindung (VI) zur Aufnahme eines H⁺-Ions fähig unter Bildung einer Säure mit einem pKₐ-Wert im Bereich von 7 bis 11, vorzugsweise 8 bis 10,5, gemessen in Wasser bei 25°C.

Erfindungsgemäß weist Verbindung (VI) eine relative Nukleophilie, gemessen in Methylperchlorat/Methanol bei 25°C gemäß Koskikallo, Acta Chem. Scand. 23 (1969) Seiten 1477-1489 und bestimmt gemäß Seite 1487-1488, im Bereich von 3,4 bis 4,7 vorzugsweise 3,6 bis 4,6, im Falle von Sauerstoff als nukleophilem Atom, im Bereich von 4,5 bis 5,8 vorzugsweise 4,8 bis 5,7 im Falle von Stickstoff als nukleophilem Atom und im Bereich von 5,5 bis 6,8 vorzugsweise 5,8 bis 6,7 im Falle von Schwefel als nukleophilem Atom auf.

Im Falle von Sauerstoff als nukleophilem Atom von (VI) kommen vorteilhaft Phenolate in Betracht, wobei das aromatische Ringsystem des Phenolats einfach oder mehrfach, wie zwei- oder dreifach substituiert sein kann, beispielsweise durch eine C₁- bis C₄-Alkylgruppe, wie Methyl, Ethyl, n-Propyl, i-Propyl, n-Butyl, i-Butyl, s-Butyl, t-Butyl, durch Halogen, wie Fluor, Chlor, Brom, Jod, durch eine Nitrogruppe, durch eine Estergruppe, durch eine Carbonylgruppe oder durch eine Aminogruppe.

Im Falle von Stickstoff als nukleophilem Atom von (VI) kommen vorteilhaft Verbindungen mit der strukturellen Einheit

(R⁴ R⁵ N) -

mit
- R⁴:: Rest einer organischen, aliphatischen, arylaliphatischen oder arylischenSäure, vorzugsweise Carbonsäure- oder Sulfonsäure-gruppe,
wobei der
Rest R⁴ substituiert sein kann wie bereits oben für Phenolatbeschrieben,
- R⁵:: Rest einer organischen, aliphatischen, arylaliphatischen oder arylischen Säure, vorzugsweise Carbonsäure- oder Sulfonsäure-gruppe, oderWasserstoff oder eine C₁- bis C₄-Alkylgruppen, wie Methyl, Ethyl, n-Propyl, i-Propyl, n-Butyl, i-Butyl, s-Butyl, t-Butyl,wobei der Rest R⁵ substituiert sein kann wie bereits oben fürPhenolat beschrieben
wobei R⁴ und R⁵ außerhalb des in der obigen Formel genannten Stickstoffs miteinander verknüpft sein können, beispielsweise über eine Alkylen-,Alkylarylen- oder eine Arylenbrücke, vorzugsweise über eine Arylenbrücke,in Betracht.

In einer bevorzugten Ausführungsform kann als Nukleophil (VI) ein Lactam-Anion eingesetzt werden der allgemeinen Formel 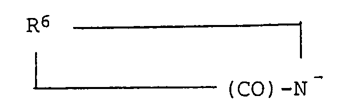 wobei
- R⁶: einen Alkylenrest mit 3, 4, 5, 6, 7, 8, 9, 10, 11 zu dem Ringsystem gehörigen Kohlenstoffatomen darstellt, wobei der Rest R⁶ substituiertsein kann wie bereits oben für Phenolat beschrieben.

In einer bevorzugten Ausführungsform kann als Nukleophil (VI) Caprolactam-Anion eingesetzt werden.

In einer weiteren besonders bevorzugten Ausführungsform kann als Nukleophil (VI) Benzolsulfonamid-Anion eingesetzt werden.

In einer weiteren besonders bevorzugten Ausführungsform kann als Nukleophil (VI) Phthalimid-Anion eingesetzt werden.

In einer weiteren besonders bevorzugten Ausführungsform kann als Nukleophil (VI) Phenolat eingesetzt werden.

Zum Ausgleich der negativen Ladung des anionischen Nukleophils (VI) kann man dieses zusammen mit einem oder mehreren Kationen, vorzugsweise ausgewählt aus der Gruppe bestehend aus Lithium, Natrium, Kalium, Rubidium, Cäsium, Magnesium, Calcium, insbesondere bestehend aus Lithium, Natrium, Kalium, Magnesium, Calcium, besonders bevorzugt bestehend aus Natrium und Kalium einsetzen.

Erfindungsgemäß versetzt man Mischung (V) mit Nukleophil (VI) in einer Menge im Bereich-von 0,01 bis 10 Mol pro Mol an Amin (IV) in Mischung (V).

Vorteilhaft kann die Menge an Nukleophil (VI) mindestens 0,05, vorzugsweise mindestens 0,1 Mol, insbesondere 0,2 Mol pro Mol an Amin (IV) in Mischung (V) betragen.

Vorteilhaft kann die Menge an Nukleophil (VI) höchstens 1, insbesondere höchstens 0,8, besonders bevorzugt höchstens 0,5 Mol pro Mol an Amin (IV) in Mischung (V) betragen.

Die Zugabe von Nukleophil (VI) zu Mischung (V) kann in an sich bekannter Weise, beispielsweise in üblichen Mischapparaturen, wie Behälter, Produktleitungen und Mischeinrichtungen, erfolgen unter Erhalt einer Mischung (VII).

Die Zugabe von Nukleophil (VI) zu Mischung (V) kann vor der Zuführung der Mischung (VII) in eine Destillationsvorrichtung zur Abtrennung gemäß Schritt b) erfolgen.

Dabei haben sich durchschnittliche mittlere Kontaktzeiten von Mischung (V) und Nukleophil (VI) vor der Zuführung in eine Destillationsvorrichtung von mindestens 5 Minuten, vorzugsweise mindestens 10 Minuten als vorteilhaft erwiesen.

Im allgemeinen haben sich deutlich längere Verweilzeiten, wie mehr als 30 Minuten, als technisch zunehmend vorteilhaft erwiesen, wobei erheblich längere Verweilzeiten zunehmend unwirtschaftlicher werden.

Nach bisherigen Beobachtungen haben sich durchschnittliche mittlere Kontaktzeiten von Mischung (V) und Nukleophil (VI) vor der Zuführung in eine Destillationsvorrichtung von höchstens 360 Minuten, vorzugsweise höchstens 180 Minuten, insbesondere höchstens 120 Minuten als vorteilhaft erwiesen.

Dabei kommen vorteilhaft Temperaturen von höchstens 200°C, vorzugsweise höchstens 190°C, insbesondere höchstens 180°C in Betracht.

Dabei kommen vorteilhaft Temperaturen von mindestens 50°C, vorzugsweise mindestens 100°C, insbesondere mindestens 140°C in Betracht.

Ebenso ist es möglich, Mischung (V) und Nukleophil (VI) einer solchen Vorrichtung getrennt zuzuführen und die Umsetzung von Mischung (V) mit Nukleophil (VI) und die Abtrennung gemäß Schritt in einem Verfahrensschritt durchzuführen. Dabei kann Nukleophil (VI) auf den Kopf, über die gesamte Höhe auf eine der Trennstufen oder in den Sumpf der Destillationsvorrichtung zugegeben werden.

Erfindungsgemäß destilliert man Aminonitril (I), Diamin (II), Dinitril (III) oder deren Gemische von Mischung (VII) bei einem Druck von höchstens 100 kPa, vorzugsweise höchstens 10 kPa ab.

Erfindungsgemäß destilliert man Aminonitril (I) Diamin (II), Dinitril (III) oder deren Gemische von Mischung (VII) bei einem Druck von mindestens 0,1 kPa, vorzugsweise mindestens 0,5 kPa ab.

Dabei kommen vorteilhaft Temperaturen von höchstens 200°C, vorzugsweise höchstens 190°C, insbesondere höchstens 180°C in Betracht.

Dabei kommen vorteilhaft Temperaturen von mindestens 50°C, vorzugsweise mindestens 100°C, insbesondere mindestens 140°C in Betracht.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, vorzugsweise in der in Ullmann's Encyclopedia of Industrial Chemistry, 5. Ed., Vol. A25, VCH Verlagsgesellschaft mbH, Weinheim, S. 215-226, insbesondere in den Absätzen 6.1.2.2.4. und 6.1.2.2.8.beschriebenen Ausgestaltung, wie Siebbodenkolonnen, Glockenbodenkolonnen, Packungskolonnen, Füllkörperkolonnen oder Seitenabzugskolonnen oder verfahrenstechnische Varianten solcher Apparaturen.

Die Destillation kann man in mehreren, wie 2 oder 3 Kolonnen, vorteilhaft einer einzigen Kolonne durchführen.

Setzt man in einer bevorzugten Ausführungsform als Mischung (V) ein Gemisch aus einem Aminonitril (I), insbesondere 6-Aminocapronitril, und einem Amin (IV) ein, so kann vorteilhaft in Schritt b) Aminonitril (I) als Kopfprodukt erhalten werden.

Setzt man in einer bevorzugten Ausführungsform als Mischung (V) ein Gemisch aus einem Aminonitril (I), insbesondere 6-Aminocapronitril, Diamin (II), insbesondere Hexamethylendiamin, und Amin (IV) ein, so kann vorteilhaft in Schritt b) Aminonitril (I), Diamin (II) oder deren Gemische, als Kopfprodukt erhalten werden.

Setzt man in einer bevorzugten Ausführungsform als Mischung (V) ein Gemisch aus einem Aminonitril (I), insbesondere 6-Aminocapronitril, Dinitril (III), insbesondere Adipodinitril, und Amin (IV) ein, so kann vorteilhaft in Schritt b) Aminonitril (I), Dinitril (III) oder deren Gemische, als Kopfprodukt erhalten werden.

Erfindungsgemäß erhält man in Schritt b) ein Sumpfprodukt.

Erfindungsgemäß destilliert man von diesem Sumpfprodukt Aminonitril (I), Diamin (II), Dinitril (III) oder deren Gemische bei einer Temperatur ab, die niedriger ist als die in Schritt b) gewählte, unter dem sich bei dieser Temperatur unter Destillationsbedingungen einstellenden Druck.

Vorteilhaft kommt eine Temperatur in Betracht, die um mindestens 1°C, vorzugsweise mindestens 5°C, insbesondere mindestens 20°C, insbesondere bevorzugt mindestens 50°C niedriger ist als die in Schritt b) gewählte.

Vorteilhaft kommt eine Temperatur in Betracht, die um höchstens 200°C, vorzugsweise höchstens 140°C, insbesondere höchstens 130°C niedriger ist als die in Schritt b) gewählte.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, vorzugsweise in der in Ullmann's Encyclopedia of Industrial Chemistry, 5. Ed., Vol. A25, VCH Verlagsgesellschaft mbH, Weinheim, S. 215-226, insbesondere in den Absätzen 6.1.2.2.4. und 6.1.2.2.8.beschriebenen Ausgestaltung, wie Siebbodenkolonnen, Glockenbodenkolonnen, Packungskolonnen, Füllkörperkolonnen oder Seitenabzugskolonnen oder verfahrenstechnische Varianten solcher Apparaturen oder eine einfache Verdampfung.

Die Destillation kann man in mehreren, wie 2 oder 3 Kolonnen, vorteilhaft einer einzigen Kolonne durchführen.

Setzt man in einer bevorzugten Ausführungsform als Mischung (V) ein Gemisch aus einem Aminonitril (I), insbesondere 6-Aminocapronitril, und einem Amin (IV) ein, so kann vorteilhaft in Schritt c) als Wertprodukt Aminonitril (I) als Kopfprodukt erhalten werden.

Setzt man in einer bevorzugten Ausführungsform als Mischung (V) ein Gemisch aus einem Aminonitril (I), insbesondere 6-Aminocapronitril, Diamin (II), insbesondere Hexamethylendiamin, und Amin (IV) ein, so kann vorteilhaft in Schritt c) als Wertprodukt Aminonitril (I), Diamin (II) oder deren Gemische, als Kopfprodukt erhalten werden.

Setzt man in einer bevorzugten Ausführungsform als Mischung (V) ein Gemisch aus einem Aminonitril (I), insbesondere 6-Aminocapronitril, Dinitril (III), insbesondere Adipodinitril, und Amin (IV) ein, so kann vorteilhaft in Schritt c) als Wertprodukt Aminonitril (I), Dinitril (III) oder deren Gemische, als Kopfprodukt erhalten werden.

In einer besonders bevorzugten Ausgestaltung dieser bevorzugten Ausführungsform kann vorteilhaft in Schritt b) ein Aminonitril (I), insbesondere 6-Aminocapronitril, als Kopfprodukt und in Schritt c) als Wertprodukt ein Dinitril (III), insbesondere Adipodinitril, als Kopfprodukt erhalten werden.

In einer insbesondere bevorzugten Ausführungsform dieser besonders bevorzugten Ausführungsform kann in einem weiteren Destillationsschritt zwischen Schritt b) und Schritt c) ein Kopfprodukt abgetrennt werden, das in einer besonders vorteilhafter Ausgestaltungsform dieser Ausführungsform in Schritt a) zurückgeführt werden kann.

Das in Schritt c) erhaltene Kopfprodukt kann vorteilhaft der gleichen Verwendung wie das in Schritt b) erhaltene Kopfprodukt zugeführt werden.

Das in Schritt c) erhaltene Kopfprodukt kann insbesondere in Schritt a) des erfindungsgemäßen Verfahrens zurückgeführt werden.

Aminonitrile (I), Diamine (II) und Dinitrile (III) stellen Vorstufen zur Herstellung von technisch wichtigen Polyamiden, wie Polyamid 6 (Nylon 6) oder Polyamid 6.6 (Nylon 6.6) dar.

Vorteilhaft können dabei die Aminonitrile (I), vorzugsweise 6-Aminocapronitril, insbesondere die in Schritt b) erhaltenen, direkt zu Polyamiden, vorzugsweise im Falle von 6-Aminocapronitril als Aminonitril (I) zu Polyamid 6 (Nylon 6), umgesetzt werden.

Vorteilhaft können dabei die Dinitrile (III), vorzugsweise Adipodinitril, insbesondere die in Schritt b) erhaltenen, zusammen mit einem Diamin, vorzugsweise einem Diamin (II), insbesondere Hexamethylendiamin, direkt zu Polyamiden, vorzugsweise im Falle von Adipodinitril als Dinitril (III) zu Polyamid 6.6 (Nylon 6.6), umgesetzt werden.

Weiterhin können Aminonitrile (I), insbesondere die in Schritt b) erhaltenen, zu Lactamen, vorzugsweise 6-Aminocapronitril zu Caprolactam, umgesetzt werden. Solche Lactame stellen Vorstufen zur Herstellung von technisch wichtigen Polyamiden, wie Polyamid. 6 (Nylon 6) dar.

Weiterhin kann das in Schritt c) erhaltene Kopfprodukt teilweise oder vollständig einem Verfahren zur Herstellung eines Aminonitrils, eines Diamins oder deren Gemische durch teilweise oder vollständige Hydrierung eines Dinitrils zugeführt werden, insbesondere im Falle von 6-Aminocapronitril, Hexamethylendiamin oder deren Gemische als Kopfprodukt in Schritt c) einem Verfahren zur teilweisen oder vollständigen Hydrierung von.Adipodinitril unter Erhalt von 6-Aminocapronitril, Hexamethylendiamin oder deren Gemische sowie gegebenenfalls von nicht umgesetztem Adipodinitril.

In einer bevorzugten Ausführungsform kann das in Stufe c) erhaltene Sumpfprodukt einer weiteren Destillation, insbesondere mittels eines Dünnschichtverdampfers, unter Erhalt von weiterem Kopfprodukt als Wertprodukt unterzogen werden.

### Beispiele

### Beispiel 1

Gemäß Schritt a) wurden 110 g/h eines 6-ACN mit einem THA-Gehalt von 420 Gew.-ppm, bezogen auf die Summe aus 6-ACN und THA, in einen Behälter gepumpt, in dem durch Zugabe eine 20 Gew.-%-igen wässrigen Lösung von Kalium-Phthalimid eine Konzentration von 130 Gew.-ppm, bezogen auf Gesamtgewicht der Mischung, eingestellt wurde. Die Temperatur in diesem Behälter betrug 170°C bei einer mittleren Verweilzeit von 1,7 Stunden.

Gemäß Schritt b) wurde die Mischung in eine Destillationsvorrichtung mit 35 theoretischen Trennstufen gepumpt und dort bei einem Kopfdruck von 4 kPa, einer Sumpftemperatur von 144°C und einem Rücklaufverhältnis von 1,7 destilliert. Als Kopfprodukt wurden 106 g/h eines 6-ACN mit einem THA-Gehalt von 37 Gew.-ppm, bezogen auf Gesamtgewicht der Mischung, erhalten.

Gemäß Schritt c) wurde das Sumpfprodukt in einem Behälter bei 90°C und 0,1 kPa teilweise verdampft, der Dampf, der als Wertprodukt 6-ACN enthielt, kondensiert und in den Behälter gemäß Schritt a) zurückgeführt. Das in Schritt c) erhaltene Sumpfprodukt wurde verworfen.

Die Ausbeute an in Schritt b) erhaltenem 6-ACN betrug 97 %, bezogen auf das in Schritt a) eingesetzte 6-ACN.

## Patentansprüche

1. Verfahren zur Reduzierung des Gehalts an einem aliphatischen, einfach ungesättigten Amin (IV) in einer Mischung (V) enthaltend ein aliphatisches Aminonitril (I) mit 4 bis 12 C-Atomen oder ein aliphatisches Diamin (II) mit 4 bis 12 C-Atomen oder ein aliphatisches Dinitril (III) mit 4 bis 12 C-Atomen oder deren Gemische und Amin (IV), wobei man
a) Mischung (V) mit einem anionischen Nukleophil (VI),
das ein nukleophiles Atom ausgewählt aus der Gruppe bestehend aus Sauerstoff, Stickstoff und Schwefel aufweist,
das zur Aufnahme eines H⁺-Ions fähig ist unter Bildung einer Säure mit einem pKₐ-Wert im Bereich von 7 bis 11, gemessen in Wasser bei 25°C, und
das eine relative Nukleophilie, gemessen in Methylperchlorat/Methanol bei 25°C,
im Bereich von 3,4 bis 4,7 im Falle von Sauerstoff als nukleophilem Atom,
im Bereich von 4,5 bis 5,8 im Falle von Stickstoff als nukleophilem Atom und
im Bereich von 5,5 bis 6,8 im Falle von Schwefel als nukleophilem Atom
aufweist,
in einer Menge im Bereich von 0,01 bis 10 Mol pro Mol an Amin (IV) in Mischung (V) bei einer Temperatur im Bereich von 50 bis 200°C unter Erhalt einer Mischung (VII) umsetzt,
b) Aminonitril (I) oder Diamin (II) oder Dinitril (III) oder deren Gemische von Mischung (VII) bei einer Temperatur im Bereich von 50 bis 200°C und einem Druck im Bereich von 0,1 bis 100 kPa abdestilliert unter Erhalt eines Sumpfproduktes (VIII),
**dadurch gekennzeichnet, dass** man
c) von Sumpfprodukt (VIII) ein Aminonitril (I) oder Diamin (II) oder Dinitril (III) oder deren Gemische bei einer Temperatur, die niedriger ist als die in Schritt b) gewählte, abdestilliert.

2. Verfahren nach Anspruch 1, wobei man als Aminonitril (I) ein aliphatisches Aminonitril mit 4 bis 12 C-Atomen ausgewählt aus der Gruppe bestehend aus 4-Aminobutyronitril, 5-Aminovaleronitril, 2-Methyl-5-aminovaleronitril, 6-Aminocapronitril, 12-Aminododecanitril einsetzt.

3. Verfahren nach Anspruch 1, wobei man als Diamin (II) ein aliphatisches Diamin mit 4 bis 12 C-Atomen ausgewählt aus der Gruppe bestehend aus 1,4-Diaminobutan, 1,5-Diaminopentan, 2-Methyl-1,5-diaminopentan, 1,6-Diaminohexan, 1,12-Diaminododecan einsetzt.

4. Verfahren nach Anspruch 1, wobei man als Dinitril (III) ein aliphatisches Dinitril mit 4 bis 12 C-Atomen ausgewählt aus der Gruppe bestehend aus 1,2-Dinitriloethan, 1,3-Dinitrilopropan, 1-Methyl-1,3-dinitrilopropan, 1,4-Dinitrilobutan, 1,10-Dinitrilodecan einsetzt.

5. Verfahren nach den Ansprüchen 1 bis 4, wobei man als Amin (IV) eine Verbindung ausgewählt aus der Gruppe bestehend aus, Dihydropyrrol, Tetrahydropyridin, 3-Methyl-Tetrahydropyridin, Tetrahydroazepin, 2-Aminoazepan, N-(2-Azepano)-1,6-Diaminohexan, N-(2-Azepano)-6-Aminocapronitril und Mono-ungesättigte Cyclododecylamine einsetzt.

6. Verfahren nach den Ansprüchen 1 bis 5, wobei man als Nukleophil (VI) Benzolsulfonamid-Anion einsetzt.

7. Verfahren nach den Ansprüchen 1 bis 6, wobei man als Nukleophil (VI) Phthalimid-Anion einsetzt.

8. Verfahren nach den Ansprüchen 1 bis 7, wobei man als Nukleophil (VI) Phenolat einsetzt.

9. Verfahren nach den Ansprüchen 1 bis 8, wobei man als Nukleophil (VI) ein Lactam-Anion einsetzt der allgemeinen Formel 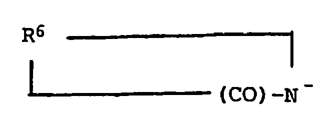 wobei R⁶ einen Alkylenrest mit 3. 4, 5, 6, 7, 8, 9, 10, 11 zu dem Ringsystem gehörenden Kohlenstoffatomen darstellt.

10. Verfahren nach den Ansprüchen 1 bis 9, wobei man als Nukleophil (VI) Capolactam-Anion einsetzt.

11. Verfahren nach den Ansprüchen 1 bis 10, wobei man anionisches Nukleophil (VI) zusammen mit einem Kation ausgewählt aus der Gruppe bestehend aus Lithium, Natrium, Kalium, Magnesium und Calcium einsetzt.

12. Verfahren nach den Ansprüchen 1 bis 11, wobei man als Mischung (V) ein Gemisch aus Aminonitril (I) und Amin (IV) einsetzt unter Erhalt von Aminonitril (I) in Schritt b) und c).

13. Verfahren nach Anspruch 12, wobei man das in Schritt c) erhaltene Aminonitril (I) in Schritt a) zurückführt.

14. Verfahren nach den Ansprüchen 1 bis 11, wobei man als Mischung (V) ein Gemisch aus Aminonitril (I), Diamin (II) und Amin (IV) einsetzt unter Erhalt von Aminonitril (I), Diamin (II) oder deren Gemische in Schritt b) und Aminonitril (I), Diamin (II) oder deren Gemische in Schritt c).

15. Verfahren nach Anspruch 14, wobei man das in Schritt c) erhaltene Aminonitril (I), Diamin (II) oder deren Gemische in Schritt a) zurückführt.

16. Verfahren nach den Ansprüchen 1 bis 11, wobei man als Mischung (V) ein Gemisch aus Aminonitril (I), Dinitril (III) und Amin (IV) einsetzt unter Erhalt von Aminonitril (I), Dinitril (III) oder deren Gemische in Schritt b) und Aminonitril (I), Dinitril (III) oder deren Gemische in Schritt c).

17. Verfahren nach Anspruch 16, wobei man das in Schritt c) erhaltene Aminonitril (I), Dinitril (III) oder deren Gemische in Schritt a) zurückführt.

18. Verfahren nach Anspruch 16 oder 17, wobei man in Schritt b) ein Aminonitril (II) und in Schritt c) ein Dinitril (III) erhält.

19. Verfahren nach den Ansprüchen 1 bis 18, wobei man das in Schritt c) erhaltene Kopfprodukt teilweise oder vollständig einem Verfahren zur Herstellung eines Aminonitrils, eines Diamins oder deren Gemische durch teilweise oder vollständige Hydrierung eines Dinitrils zuführt.

20. Verfahren nach den Ansprüchen 1 bis 19, wobei man in Schritt b) 6-Aminocapronitril als Aminonitril (I) erhält und dieses 6-Aminocapronitril direkt zu Polyamid 6 umsetzt.

21. Verfahren nach den Ansprüchen 1 bis 19, wobei man in Schritt b) 6-Aminocapronitril als Aminonitril (I) erhält und dieses 6-Aminocapronitril zu Caprolactam zyklisiert.

22. Verfahren nach den Ansprüchen 1 bis 19, wobei man in Schritt b) Adipodinitril als Dinitril (III) erthält und dieses Adipodinitril zusammen mit Hexamethylendiamin direkt zu Polyamid 6.6 umsetzt.

## Claims

1. A process for reducing the level of an aliphatic monounsaturated amine (IV) in a mixture (V) comprising an aliphatic aminonitrile (I) having from 4 to 12 carbon atoms or an aliphatic diamine (II) having from 4 to 12 carbon atoms or an aliphatic dinitrile (III) having from 4 to 12 carbon atoms or mixtures thereof as well as said amine (IV), which process comprises
a) reacting said mixture (V) with an anionic nucleophile (VI)
which contains a nucleophilic atom selected from the group consisting of oxygen, nitrogen and sulfur,
which is capable of taking up an H⁺ ion to form an acid having a pKₐ value in the range from 7 to 11, as measured in water at 25°C, and
which has a relative nucleophilicity, as measured in methyl perchlorate/methanol at 25°C,
in the range from 3.4 to 4.7 when said nucleophilic atom is oxygen,
in the range from 4.5 to 5.8 when said nucleophilic atom is nitrogen, and
in the range from 5.5 to 6.8 when said nucleophilic atom is sulfur,
in an amount in the range from 0.01 to 10 mol per mole of said amine (IV) in said mixture (V) at a temperature in the range from 50 to 200°C to obtain a mixture (VII),
b) distilling said aminonitrile (I) or said diamine (II) or said dinitrile (III) or mixtures thereof from said mixture (VII) at a temperature in the range from 50 to 200°C and at a pressure in the range from 0.1 to 100 kPa to obtain a bottom product (VIII),
wherein
c) an aminonitrile (I) or diamine (II) or dinitrile (III) or a mixture thereof is distilled from said bottom product (VIII) at a temperature which is lower than that chosen in step b).

2. The process according to claim 1 wherein said aminonitrile (I) is an aliphatic aminonitrile having from 4 to 12 carbon atoms and selected from the group consisting of 4-aminobutyronitrile, 5-aminovaleronitrile, 2-methyl-5-aminovaleronitrile, 6-aminocapronitrile and 12-aminododecanenitrile.

3. The process according to claim 1 wherein said diamine (II) is an aliphatic diamine having from 4 to 12 carbon atoms and selected from the group consisting of 1,4-diaminobutane, 1,5-diaminopentane, 2-methyl-1,5-diaminopentane, 1,6-diaminohexane and 1,12-diaminododecane.

4. The process according to claim 1 wherein said dinitrile (III) is an aliphatic dinitrile having from 4 to 12 carbon atoms and selected from the group consisting of 1,2-dinitriloethane, 1,3-dinitrilopropane, 1-methyl-1,3-dinitrilopropane, 1,4-dinitrilobutane and 1,10-dinitrilodecane.

5. The process according to any of claims 1 to 4 wherein said amine (IV) is a compound selected from the group consisting of dihydropyrrole, tetrahydropyridine, 3-methyltetrahydropyridine, tetrahydroazepine, 2-aminoazepan, N-(2-azepano)-1,6-diaminohexane, N-(2-azepano)-6-aminocapronitrile and monounsaturated cyclododecylamines.

6. The process according to any of claims 1 to 5 wherein said nucleophile (VI) is benzenesulfonamide anion.

7. The process according to any of claims 1 to 6 wherein said nucleophile (VI) is phthalimide anion.

8. The process according to any of claims 1 to 7 wherein said nucleophile (VI) is phenate.

9. The process according to any of claims 1 to 8 wherein said nucleophile (VI) is a lactam anion of the general formula 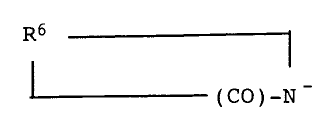 where R⁶ is an alkylene radical having 3, 4, 5, 6, 7, 8, 9, 10 or 11 carbon atoms belonging to the ring system.

10. The process according to any of claims 1 to 9 wherein said nucleophile (VI) is caprolactam anion.

11. the process according to any of claims 1 to 10 wherein said anionic nucleophile (VI) is used together with a cation selected from the group consisting of lithium, sodium, potassium, magnesium and calcium.

12. The process according to any of claims 1 to 11 wherein said mixture (V) is a mixture of said aminonitrile (I) and said amine (IV) to obtain said aminonitrile (I) in steps b) and c).

13. The process according to claim 12 wherein said aminonitrile (I) obtained in step c) is recycled into step a).

14. The process according to any of claims 1 to 11 wherein said mixture (V) is a mixture of said aminonitrile (I), said diamine (II) and said amine (IV) to obtain said aminonitrile (I), said diamine (II) or mixtures thereof in step b) and said aminonitrile (I), said diamine (II) or mixtures thereof in step c).

15. The process according to claim 14 wherein said aminonitrile (I) or said diamine (II) obtained in step c) or mixtures thereof are recycled into step a).

16. The process according to any of claims 1 to 11 wherein said mixture (V) is a mixture of said aminonitrile (I), said dinitrile (III) and said amine (IV) to obtain said aminonitrile (I), said dinitrile (III) or mixtures thereof in step b) and said aminonitrile (I), said dinitrile (III) or mixtures thereof in step c).

17. The process according to claim 16 wherein said aminonitrile (I) or said dinitrile (III) obtained in step c) or mixtures thereof are recycled into step a).

18. The process according to claim 16 or 17 wherein an aminonitrile (II) is obtained in step b) and a dinitrile (III) is obtained in step c).

19. The process according to any of claims 1 to 18 wherein the top product obtained in step c) is wholly or partly fed into a process for producing an aminonitrile, a diamine or mixtures thereof by partial or full hydrogenation of a dinitrile.

20. The process according to any of claims 1 to 19 wherein said aminonitrile (I) obtained in step b) is 6-aminocapronitrile and this 6-aminocapronitrile is directly converted into nylon-6.

21. The process according to any of claims 1 to 19 wherein said aminonitrile (I) obtained in step b) is 6-aminocapronitrile and this 6-aminocapronitrile is cyclized to caprolactam.

22. The process according to any of claims 1 to 19 wherein said dinitrile (III) obtained in step b) is adiponitrile and this adiponitrile is directly converted with hexamethylenediamine into nylon-6,6.

## Revendications

1. Procédé de réduction de la teneur en une amine aliphatique insaturée une fois (IV) dans un mélange (V) contenant un aminonitrile aliphatique (I) comportant 4 à 12 atomes de C ou une diamine aliphatique (II) comportant 4 à 12 atomes de C ou un dinitrile aliphatique (III) comportant 4 à 12 atomes de C ou leurs mélanges et de l'amine (IV), dans lequel
a) on fait réagir à une température de l'ordre de 50 à 200°C un mélange (V) avec un nucléophile anionique (VI), qui présente un atome nucléophile choisi parmi le groupe constitué de l'oxygène, de l'azote et du soufre, qui est capable de capter un ion H⁺ avec formation d'un acide ayant une valeur de pKₐ de l'ordre de 7 à 11, mesurée dans l'eau à 25°C, et
qui présente une nucléophilie relative, mesurée dans du perchlorate de méthyle/méthanol à 25°C,
de l'ordre de 3,4 à 4,7 dans le cas d'oxygène comme atome nucléophile,
de l'ordre de 4,5 à 5,8 dans le cas d'azote comme atome nucléophile, et
de l'ordre de 5,5 à 6,8 dans le cas de soufre comme atome nucléophile,
en une quantité de l'ordre de 0,01 à 10 moles par mole d'amine (IV) dans le mélange (V), avec obtention d'un mélange (VII),
b) on sépare du mélange (VII) par distillation à une température de l'ordre de 50 à 200°C et à une pression de l'ordre de 0,1 à 100 kPa de l'aminonitrile (I) ou de la diamine (II) ou du dinitrile (III) ou leurs mélanges, avec obtention d'un produit de fond de colonne (VIII),
**caractérisé en ce que**
c) on sépare par distillation du produit de fond de colonne (VIII) un aminonitrile (I) ou de la diamine (II) ou du dinitrile (III) ou leurs mélanges à une température qui est plus basse que celle choisie dans l'étape b).

2. Procédé suivant la revendication 1, dans lequel, comme aminonitrile (I), on met en oeuvre un aminonitrile aliphatique comportant 4 à 12 atomes de C choisi parmi le groupe constitué de 4-aminobutyronitrile, de 5-aminovaléronitrile, de 2-méthyl-5-aminovaléronitrile, de 6-aminocapronitrile, de 12-aminododécanitrile.

3. Procédé suivant la revendication 1, dans lequel, comme diamine (II), on met en oeuvre une diamine aliphatique comportant 4 à 12 atomes de C choisie parmi le groupe constitué de 1,4-diaminobutane, de 1,5-diaminopentane, de 2-méthyl-1,5-diaminopentane, de 1,6-diaminohexane, de 1,12-diaminododécane.

4. Procédé suivant la revendication 1, dans lequel, comme dinitrile (III), on met en oeuvre un dinitrile aliphatique comportant 4 à 12 atomes de C choisi parmi le groupe constitué de 1,2-dinitriloéthane, de 1,3-dinitrilopropane, de 1-méthyl-1,3-dinitrilopropane, de 1,4-dinitrilobutane, de 1,10-dinitrilodécane.

5. Procédé suivant les revendications 1 à 4, dans lequel, comme amine (IV), on met un oeuvre un composé choisi parmi le groupe constitué de dihydropyrrole, de tétrahydropyridine, de 3-méthyl-tétrahydropyridine, de tétrahydroazépine, de 2-aminoazépan, de N-(2-azépano)-1,6-diaminohexane, de N-(2-azépano)-6-aminocapronitrile et de cyclododécylamines monoinsaturées.

6. Procédé suivant les revendications 1 à 5, dans lequel, comme nucléophile (VI), on met en oeuvre un anion de benzènesulfonamide.

7. Procédé suivant les revendications 1 à 6, dans lequel, comme nucléophile (VI), on met en oeuvre un anion de phtalimide.

8. Procédé suivant les revendications 1 à 7, dans lequel, comme nucléophile (VI), on met en oeuvre un phénolate.

9. Procédé suivant les revendications 1 à 8, dans lequel, comme nucléophile (VI), on met en oeuvre un anion de lactame de la formule générale : 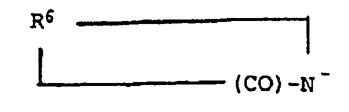 dans laquelle R⁶ représente un radical alkylène comportant 3, 4, 5, 6, 7, 8, 9, 10, 11 atomes de carbone appartenant au système cyclique.

10. Procédé suivant les revendications 1 à 9, dans lequel, comme nucléophile (VI), on met en oeuvre un anion de caprolactame.

11. Procédé suivant les revendications 1 à 10, dans lequel on met en oeuvre un nucléophile anionique (VI) conjointement à un cation choisi parmi le groupe constitué du lithium, du sodium, du potassium, du magnésium et du calcium.

12. Procédé suivant les revendications 1 à 11, dans lequel, comme mélange (V), on met en oeuvre un mélange d'aminonitrile (I) et d'amine (IV), avec obtention d'aminonitrile (I) dans les étapes b) et c) .

13. Procédé suivant la revendication 12, dans lequel on recycle dans l'étape a) l'aminonitrile (I) obtenu dans l'étape c).

14. Procédé suivant les revendications 1 à 11, dans lequel, comme mélange (V), on met en oeuvre un mélange d'aminonitrile (I), de diamine (II) et d'amine (IV), avec obtention d'aminonitrile (I), de diamine (II) ou de leurs mélanges dans l'étape b) et d'aminonitrile (I), de diamine (II) ou de leurs mélanges dans l'étape c).

15. Procédé suivant la revendication 14, dans lequel on recycle dans l'étape a) l'aminonitrile (I), la diamine (II) ou leurs mélanges obtenus dans l'étape c).

16. Procédé suivant les revendications 1 à 11, dans lequel, comme mélange (V), on met en oeuvre un mélange d'aminonitrile (I), de dinitrile (III) et d'amine (IV), avec obtention d'aminonitrile (I), de dinitrile (III) ou de leurs mélanges dans l'étape b) et d'aminonitrile (I), de dinitrile (III) ou de leurs mélanges dans l'étape c).

17. Procédé suivant la revendication 16, dans lequel on recycle dans l'étape a) l'aminonitrile (I), le dinitrile (III) ou leurs mélanges obtenus dans l'étape c).

18. Procédé suivant la revendication 16 ou 17, dans lequel on obtient dans l'étape b) un aminonitrile (II) et dans l'étape c) un dinitrile (III).

19. Procédé suivant les revendications 1 à 18, dans lequel on amène le produit de tête obtenu dans l'étape c) partiellement ou totalement à un procédé de préparation d'un aminonitrile, d'une diamine ou de leurs mélanges, par hydrogénation partielle ou totale d'un dinitrile.

20. Procédé suivant les revendications 1 à 19, dans lequel on obtient dans l'étape b) du 6-aminocapronitrile comme aminonitrile (I) et on fait réagir directement ce 6-aminocapronitrile pour obtenir du polyamide 6.

21. Procédé suivant les revendications 1 à 19, dans lequel on obtient dans l'étape b) du 6-aminocapronitrile comme aminonitrile (I) et on cyclise ce 6-aminocapronitrile en caprolactame.

22. Procédé suivant les revendications 1 à 19, dans lequel on obtient dans l'étape b) de l'adipodinitrile comme dinitrile (III) et on fait réagir cet adipodinitrile conjointement à de l'hexaméthylènediamine pour former directement du polyamide 6.6.
